# EUROPEAN PATENT SPECIFICATION

(11) **EP 3 233 790 B1**
(45) Date of publication and mention of the grant of the patent: **26.06.2019**
(21) Application number: 15817286.6
(22) Date of filing: 18.12.2015
(51) Int. Cl.: C08J 9/08, B60R 13/08

(54) **CHEMICAL BLOWING AGENT AND THERMALLY EXPANDABLE THERMOPLASTIC COMPOSITION**
CHEMISCHES TREIBMITTEL UND WÄRMEDEHNBARE THERMOPLASTISCHE ZUSAMMENSETZUNG
AGENT GONFLANT CHIMIQUE ET COMPOSITION THERMOPLASTIQUE EXPANSIBLE THERMIQUEMENT

(30) Priority: 19.12.2014 EP 14199411
(43) Date of publication of application: 25.10.2017
(73) Proprietor: Sika Technology AG, 6340 Baar (CH)
(72) Inventor: BORDEANU, Nicolae, Rochester Hills, MI 48309 (US); XU-RABL, Rui, 8049 Zürich (CH); BURCKHARDT, Urs, 8049 Zürich (CH)
(74) Representative: Sika Patent Attorneys
(86) International application number: PCT/EP2015/080639
(87) International publication number: WO 2016/097365

(56) References cited:
- US-A- 3 425 964
- US-A- 5 266 133
- US-A1- 2012 156 612
- RAINER HERGES ET AL: "Design of a Neutral Macrocyclic Ionophore: Synthesis and Binding Properties for Nitrate and Bromide Anions", EUROPEAN JOURNAL OF ORGANIC CHEMISTRY, vol. 2002, no. 17, 1 September 2002 (2002-09-01), pages 3004-3014, XP055191477, ISSN: 1434-193X, DOI: 10.1002/1099-0690(200209)2002:17<3004::AID -EJOC3004>3.0.CO;2-O
- DAVID SPIVAK ET AL: "Molecular Imprinting of Carboxylic Acids Employing Novel Functional Macroporous Polymers", THE JOURNAL OF ORGANIC CHEMISTRY, vol. 64, no. 13, 1 June 1999 (1999-06-01), pages 4627-4634, XP055191478, ISSN: 0022-3263, DOI: 10.1021/jo982118s
- DIETER ENDERS ET AL: "Diastereo- and Enantioselective Synthesis of Vicinal Diamines via Aza-Michael Addition to Nitroalkenes", SYNTHESIS, vol. 1996, no. 12, 1 December 1996 (1996-12-01), pages 1443-1450, XP055191479, ISSN: 0039-7881, DOI: 10.1055/s-1996-4411
- THOMAS FRANCIS ET AL: "carbamates and 2-oxazolidinones from tertiary alcohols and isocyanates", CANADIAN JOURNAL OF CHEMISTRY, NATIONAL RESEARCH COUNCIL. OTTAWA; CA, vol. 54, 1 January 1976 (1976-01-01), pages 24-30, XP009111686,
- CHRISTIAN STOCK ET AL: "Mild and High-Yielding Molybdenum(VI) Dichloride Dioxide-Catalyzed Formation of Mono-, Di-, Tri-, and Tetracarbamates from Alcohols and Aromatic or Aliphatic Isocyanates", ADVANCED SYNTHESIS & CATALYSIS, vol. 354, no. 11-12, 13 August 2012 (2012-08-13), pages 2309-2330, XP055191480, ISSN: 1615-4150, DOI: 10.1002/adsc.201200303
- ALBRECHT BERKESSEL ET AL: "Enantiomerically Pure Isophorone Diamine [3-(Aminomethyl)-3,5,5-trimethylcyclohexyl amine]: A Chiral 1,4-Diamine Building Block Made Available on Large Scale", THE JOURNAL OF ORGANIC CHEMISTRY, vol. 71, no. 25, 1 December 2006 (2006-12-01), pages 9312-9318, XP055191481, ISSN: 0022-3263, DOI: 10.1021/jo0613737

## Description

### Technical field

The present invention relates to chemical blowing agents suitable to foam thermoplastic materials, methods for manufacturing them, their use in thermoplastic compositions, and thermally expandable thermoplastic compositions comprising these chemical blowing agents, in particular for use in baffle or reinforcement elements in automotive parts or building construction.

### Background of the invention

Thermally expandable thermoplastic compositions, normally comprising chemical blowing agents which decompose in a controlled fashion accompanied by gas release under defined high temperature conditions, are useful tools for many applications. Upon thermal activation of the blowing agent, such expandable compositions form rigid, foam-like cellular structures incorporating gas bubbles in the thermoplastic matrix and they expand volumetrically by normally several 100 %, thus efficiently filling cavities or hollow parts in objects of manufacturing, such as automotive vehicles or buildings. Often they serve the purpose of reinforcing the hollow structures or cavities without adding excessive weight, or they act as insulation material able to dampen for example noise and vibrations or as thermal insulation.

For thermally expandable thermoplastic compositions used in automotive manufacturing, i.e. for baffle and reinforcement elements in hollow car body structures, the nowadays most important chemical blowing agent is azodicarbonamide (ADCA), also called azo(bis)formamide. It has a broad decomposition temperature range adjustable between about 140 °C and 200 °C and exhibits a high decomposition gas volume of approximately 200 mL/g. Furthermore, it has a very attractive price/performance ratio. However, recently raised concerns by European legislators regarding potentially adverse health effects for this class of compounds may lead to restrictions or even a ban of their use in expandable thermoplastic compositions, which increases demand for viable alternatives.

Some alternative chemical blowing agents are already available, such as sodium bicarbonate, oxybis(benzenesulfonyl hydrazide) (OBSH), toluenesulfonyl hydrazide (TSH), p-toluenesulfonyl semicarbazide (TSSC), 5-phenyl tetrazole (5-PT), or N-N'-dinitrosopentamethylene tetramine (DNPT). These compounds however are only useful in limited applications, as they either suffer from poor decomposition gas volume, release toxic, reactive gases such as formaldehyde, or require activation temperatures difficult to meet for baffle or reinforcement elements in automotive manufacturing.

US 3 425 864 A discloses a latent curing agent made from the reaction of carbon dioxide with a liquid polyamine and its use in a thermosetting resin to cure and foam the resin.

Therefore, there is still an unmet demand for an alternative chemical blowing agent which is commercially viable and shows at least comparable performance as ADCA regarding gas volume and decomposition temperature range and which can be easily incorporated in thermally expandable thermoplastic compositions that are useful, for example, for baffle or reinforcement elements in automotive manufacturing or building insulation.

### Summary of the invention

Thus, the object of the present invention is to provide a thermally expandable composition comprising a chemical blowing agent suitable in particular for baffle or reinforcement elements such as those used in automotive manufacturing, the chemical blowing agent exhibiting a controllable decomposition with high gas volume, suitable decomposition temperature range and non-toxic decomposition gases.

Surprisingly, this object could be achieved with the features of independent claim 1.

Such chemical blowing agents comprised in the thermally expandable compositions according to the present invention reach comparable performance as ADCA in thermally expandable thermoplastic compositions and they can be synthetically tailored, for example regarding decomposition temperature or gas volume, for very specific applications, using readily available precursor materials. Furthermore, their non-gaseous reaction products are reactive with certain thermoplastic polymers and can be used to further strengthen the polymer network of the expanded thermoplastic material in certain embodiments.

Further aspects of the present invention are object of other independent claims. Preferred embodiments of the present invention are object of the dependent claims.

### Detailed description of the invention

In a first aspect, the object of the present invention is a thermally expandable composition, comprising:
a) at least one chemical blowing agent,
b) at least one thermoplastic material,
c) optionally at least one stabilizer **S,** wherein
the at least one chemical blowing agent comprises at least one carbamate **CA** according to formula (I), 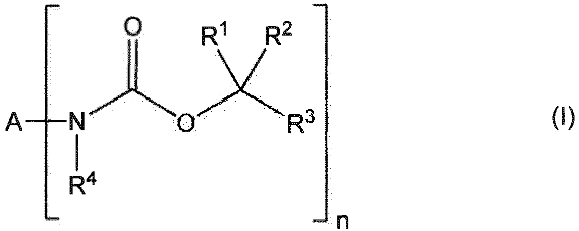 wherein
R¹ and R² independently represent linear or branched monovalent hydrocarbon radicals having 1 to 10 carbon atoms, preferably 1 to 5 carbon atoms, which optionally contain one or more heteroatoms, and/or C-C multiple bonds, and/or cycloaliphatic, and/or aromatic moieties, or together represent a divalent hydrocarbon radical having 4 to 10 carbon atoms, preferably 4 to 6 carbon atoms;
R³ represents a linear or branched monovalent hydrocarbon radical having 1 to 20 carbon atoms, preferably 1 to 10 carbon atoms, and optionally contains one or more heteroatoms, in particular one or more optionally substituted tertiary alkyl carbamates, and/or C-C multiple bonds, and/or cycloaliphatic, and/or aromatic moieties;
R⁴ represents a hydrogen radical or a linear or branched monovalent hydrocarbon radical having 1 to 10 carbon atoms, preferably 1 to 5 carbon atoms, which optionally contain one or more heteroatoms, and/or C-C multiple bonds, and/or cycloaliphatic, and/or aromatic moieties, or together with A represents an (n+1)-valent hydrocarbon radical having 4 to 40 carbon atoms, preferably 4 to 20 carbon atoms, which optionally contains one or more heteroatoms, and/or cycloaliphatic moieties;
A represents a linear or branched n-valent hydrocarbon radical having 1 to 20 carbon atoms, which optionally contains one or more heteroatoms, in particular in the form of ether oxygen or amine nitrogen, and optionally one or more C-C multiple bonds, and/or cycloaliphatic, and/or aromatic moieties, or together with R⁴ represents an (n+1)-valent hydrocarbon radical having 4 to 40 carbon atoms, preferably 4 to 20 carbon atoms, which optionally contains one or more heteroatoms, and/or cycloaliphatic moieties;
index n stands for a value of 1 or 2 or 3 or 4, preferably 2 or 3.

Substance names beginning with "poly" such as polyamine or polyisocyanate refer to substances that formally contain two or more of the functional groups occurring in their names, as opposed to monoamine or monoisocyanate which contain only one amine or isocyanate group per molecule, respectively. The term "polymer" includes, on the one hand, a collection of macromolecules that are uniform chemically but differ with respect to the degree of polymerization, the molecular weight and the chain length and were synthesized by a polyreaction (polymerization, polyaddition, polycondensation). On the other hand, this term also includes derivatives of such a group of macromolecules of polyreactions, i.e., compounds obtained by reactions, such as additions or substitutions of functional groups on given macromolecules, which may be chemically uniform or chemically heterogeneous. This term also includes so-called prepolymers, i.e., reactive oligomeric preadducts, whose functional groups are involved in the synthesis of macromolecules.

The term "polyurethane polymer" includes all polymers synthesized by the so-called diisocyanate polyaddition process. This also includes polymers which are almost or entirely free of urethane groups. Examples of polyurethane polymers include polyether polyurethanes, polyester polyurethanes, polyether polyureas, polyureas, polyester polyureas, polyisocyanurates and polycarbodiimides.

The term "isocyanate-functional" denotes compounds containing isocyanate groups. "Isocyanate-functional polymers" are thus polymers containing at least one isocyanate group. Similarly, the term "amine-functional" denotes compounds containing an amine functional group.

"Primary amines" are molecules having at least one primary amino group, i.e., an NH₂ group bound to an organic radical. "Secondary amines" are amines having at least one secondary amino group, i.e., an NH group bound to two organic radicals. "Tertiary alcohols" describe molecules which have a hydroxyl group bound to a tertiary carbon, i.e. a molecule with the structure HO-C(R)(R')(R"), wherein R, R', and R" are organic radicals but not hydrogen. "Tertiary alkyl carbamates" formally describes esters of carbamic acid with a tertiary alcohol and includes molecules containing this structural element, which for example also corresponds to carbamates according to formula (I).

"Molecular weight" refers to the molar mass (in grams per mole) of a molecule. The term "average molecular weight" refers to the average molar mass of an oligomer or polymer and describes the number average molar mass (Mₙ) of a mixture of oligomer or polymer molecules, typically measured by gel permeation chromatography (GPC) in THF using a polystyrene standard.

"Gas volume" or "decomposition gas volume" describes the volume (in milliliters, abbreviated "mL", unless otherwise specified) of gases which are formed when 1 gram (or an amount explicitly specified) of a blowing agent, or of a specific chemical substance comprised in a blowing agent, decomposes. The gas volume is normally measured at ambient pressure and at the temperature specified.

Carbamate **CA** comprised in the chemical blowing agent may exhibit any structure according to formula (I) to be suitable for a use in the chemical blowing agent. In other words, they all have at least one optionally substituted tertiary alkyl carbamate ester function in their molecular structure.

Preferred embodiments of carbamate **CA** comprised in the chemical blowing agent are those according to formula (I) wherein said index n equals 2 and said radical A represents a linear or branched divalent hydrocarbon radical having 2 to 10 carbon atoms and which optionally contains one or more heteroatoms, in particular in the form of ether oxygen.

Furthermore preferred are chemical blowing agents comprising carbamate **CA** according to formula (I), wherein said radicals R¹, R² and R³ each represent methyl groups. These carbamates are especially advantageous regarding high gas volume as they form isobutylene during decomposition and they are accessible by at least two different synthetic routes, which are discussed in more detail further below. Among these, particularly preferred are carbamate **CA** according to formula (I) wherein wherein said radical R¹, R² and R³ each represent methyl groups and R⁴ represents a hydrogen radical.

Another advantage of using carbamates **CA** according to formula (I) as blowing agents in certain embodiments may be the fact that amines are formed in the decomposition reaction. These amines may have catalytic properties in certain reactions or they may be incorporated in polymer cross-linking reactions, e.g. in polyurethane or epoxy systems where they can react with (poly)isocyanates or (poly)epoxides. This however depends on the respective application and has to be taken into account when designing such a system. The aspect of using carbamates **CA** in blowing agents in thermally expandable thermoplastic compositions with such polymers is another aspect of the present invention and discussed further below.

The chemical blowing agent preferably comprises carbamate **CA** with low molecular weight and/or high carbamate functionality. This ensures a high gas volume per mass unit of chemical blowing agent employed in any process. Thus, those embodiments are preferred wherein said carbamate **CA** has a molecular weight of between 131 g/mol and 750 g/mol, preferably between 131 g/mol and 500 g/mol, more preferably between 131 g/mol and 450 g/mol.

Accordingly, the chemical blowing agent preferably produces a high gas volume when decomposing in the way intended by the aspects of the present invention. This means that in preferred embodiments, the carbamate **CA** of the chemical blowing agent produces at least 140 mL, preferably at least 160 mL, more preferably at least 180 mL, most preferably at least 200 mL gas per gram carbamate **CA** when heated above its decomposition temperature until essentially all of the carbamate **CA** has decomposed. The gas volume is measured at ambient pressure (approximately 1 bar) and 23 °C gas temperature.

The chemical blowing agent is efficient, meaning that it produces gas fast enough to be suitable as blowing agent, for example to foam thermoplastic compositions. Thus, in preferred embodiments at least 50% of the carbamate **CA** comprised in the blowing agent decomposes under gas release at a temperature of between 170 °C and 230 °C within 10 min. In other preferred embodiments at least 95% of the carbamate **CA** comprised in the blowing agent decomposes under gas release at a temperature of between 170 °C and 230 °C within 20 min.

A method of manufacturing a preferred carbamate **CA** suitable for use in a chemical blowing agent as described above, comprises the reaction of an amine according to formula (II) with n equivalents di-*tert*-butyl dicarbonate,

A⁅NHR⁴]n (II)

wherein radicals A and R⁴ and index n have the same meaning as described before.

Carbamates **CA** manufactured in this way are especially preferred for the blowing agent. They have the general structure as shown in formula (Ia) with R¹, R² and R³ each representing methyl groups.

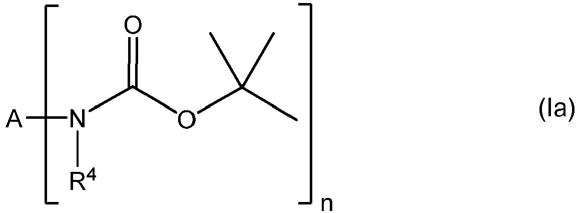

The carbamates **CA** according to formula (Ia) have the advantage that they produce an especially high amount of gas when decomposing, since per carbamate group according to (Ia), 1 molecule of CO₂ and 1 molecule of isobutylene are released. This leads to a higher gas volume and better foam structure.

This method of manufacturing carbamates **CA** suitable for the present invention uses the reaction of amines with equimolar amounts of di-*tert*-butyl dicarbonate to form the corresponding *tert*-butyl carbamate (Ia). This reaction is known to introduce *tert*-butyloxycarbonyl (BOC) groups, normally in the function as a protecting group, to amines. This is performed with well-known synthetic procedures, for example in tetrahydrofuran (THF) with triethylamine base at 25 °C.

Suitable amines of the formula (II) include linear or branched mono- or polyamines having 1 to 20 carbon atoms which optionally contain one or more heteroatoms, in particular in the form of ether oxygen or amine nitrogen, and optionally one or more C-C multiple bonds, and/or cycloaliphatic, and/or aromatic moieties. Such amines can have 1 to 4 primary and/or secondary amino groups, while polyamines with 2 or 3 primary and/or secondary amino groups are preferred.

Suitable monoamines include primary monoamines (with R⁴ = H and n = 1), such as methylamine, ethylamine, 1-propylamine, 2-propylamine, 1-butylamine, 2-butylamine, isobutylamine, 1-pentylamine, isopentylamine, 1-hexylamine, isohexylamine, 2-ethylhexylamine, 1-octylamine, 1-decylamine, 1-dodecylamine, cyclohexylamine, benzylamine, N,N-dimethylpropane-1,3-diamine, N-(2-aminoethyl)morpholine, N-methyl-N'-(2-aminoethyl)piperazine, N-(3-aminopropyl)piperidine, N-(3-aminopropyl)morpholine, 2-methoxyethylamine, 2-ethoxyethylamine, 2-n-butoxyethylamine, 2-cyclohexyloxyethylamine, 2-benzyloxyethylamine, 3-methoxypropylamine, 3-ethoxypropylamine, 3-n-butoxypropylamine, 3-cyclohexyloxypropylamine, 3-phenyloxypropylamine, 3-(2-methoxyethoxy)propylamine or 2(4)-methoxyphenylethylamine.

Suitable secondary monoamines include dimethylamine, diethylamine, di-n-propylamine, diisopropylamine, di-n-butylamine, diisobutylamine, di-n-hexylamine, diisohexylamine, di(2-ethylhexyl)amine, dicyclohexylamine, dibenzylamine, methylethylamine, methylisopropylamine, methyl-n-butylamine, methylcyclohexylamine, methylbenzylamine, pyrrolidine, piperidine, hexamethyleneimine, N-methylpiperazine, N-ethylpiperazine, morpholine or 2,6-dimethylmorpholine.

Preferred suitable primary polyamines include ethane-1,2-diamine, propane-1,2-diamine, propane-1,3-diamine, butane-1,3-diamine, butane-1,4-diamine, pentane-1,5-diamine, 2-methyl-1,5-pentanediamine (MPMD), 2-butyl-2-ethyl-1,5-pentanediamine, hexane-1,6-diamine, 2,2,4- and 2,4,4-trimethyl-1,6-hexanediamine, octane-1,8-diamine, decane-1,8-diamine, dodecane-1,12-diamine, 2,2-dimethylpropane-1,3-diamine, 4-(aminomethyl)octane-1,8-diamine, 1,2-diaminocyclohexane, 1,3-diaminocyclohexane, 1,4-diaminocyclohexane, bis(4-aminocyclohexyl)methane, bis(4-amino-3-methylcyclohexyl)-methane, 1-amino-3-aminomethyl-3,5,5-trimethylcyclohexane (isophoronedi-amine or IPDA), 2- and 4-methyl-1,3-diaminocyclohexane, 1,3-bis(aminomethyl)cyclohexane, 1,4-bis(aminomethyl)cyclohexane, 2,5(2,6)-bis(aminomethyl)bicyclo[2.2.1]heptane (NBDA), 3(4), 8(9)-bis(aminomethyl)-tricyclo[5.2.1.0^{2,6}]decane, 1,8-menthanediamine, 1,3-bis(aminomethyl)-benzene, 1,4-bis(aminomethyl)benzene, 1,4-bis(2-aminoethyl)piperazine, N,N'-bis(aminopropyl)-piperazine, N,N-bis(3-aminopropyl)methylamine, 3-oxapentane-1,5-diamine (available under the trade name Jeffamine® EDR-104, by Huntsman), 3,6-dioxaoctane-1,8-diamine (available under the trade name Jeffamine® EDR-148, by Huntsman), and 4,7-dioxadecane-1,10-diamine (available under the trade name Jeffamine® EDR-176, by Huntsman).

Preferred secondary polyamines include piperazine, N-(2-aminopropyl)piperazine, 4,4'-trimethylenedipiperidine; further suitable are N-monoalkylated or N,N'-dialkylated primary diamines as described above, whereby the alkyl groups are preferably selected from methyl, ethyl, n-propyl, isopropyl, n-butyl, 2-butyl and isobutyl, more preferably N,N'-dimethylated primary diamines, in particular 3-Dimethylaminopropylamine (DMAPA), N-methyl-ethane-1,2-diamine or N,N'-dimethyl-ethane-1,2-diamine; further suitable are N-aminopropylated primary monoamines, in particular N-methylpropane-1,3-diamine, N-butyl-propane-1,3-diamine, N-cyclohexyl-propane-1,3-diamine, N1-(3-(Dimethylamino)propyl)-1,3-diaminopropan (DMAPAPA), N-(2-ethylhexyl)-propane-1,3-diamine, N-dodecyl-propane-1,3-diamine or fatty diamines such as N-cocoalkyl-propane-1,3-diamin, N-oleyl-propane-1-3-diamine or N-soyalkyl-propane-1,3-diamine (available e.g. under the trade name Duomeen®, by Akzo Nobel).

Also suitable are aminoalcohols such as 2-aminoethanol, 2-amino-1-propanol, 1-amino-2-propanol, 3-amino-1-propanol, 5-amino-1-pentanol, 6-amino-1-hexanol, 3-aminomethyl-3,5,5-trimethyl-cyclohexanol, 2-(2-aminoethoxy)-ethanol, N-methyl-2-aminoethanol, N-butyl-2-aminoethanol, N-butyl-2-aminopropanol, diethanolamine or diisopropanolamine.

Most preferred are di- and triamines, especially diamines including ethane-1,2-diamine, propane-1,2-diamine, propane-1,3-diamine, butane-1,3-diamine, butane-1,4-diamine, pentane-1,5-diamine, 2-methyl-1,5-pentanediamine (MPMD), octane-1,8-diamine, decane-1,8-diamine, dodecane-1,12-diamine, 2,2-dimethylpropane-1,3-diamine, 4-(aminomethyl)octane-1,8-diamine, piperazine, 3-oxapentane-1,5-diamine (available under the trade name Jeffamine® EDR-104, by Huntsman), 3,6-dioxaoctane-1,8-diamine (available under the trade name Jeffamine® EDR-148, by Huntsman), and 4,7-dioxadecane-1,10-diamine (available under the trade name Jeffamine® EDR-176, by Huntsman).

As mentioned before, carbamates **CA** according to formula (Ia) are especially advantageous due to their high gas volume, while the formed gas is essentially non-reactive and non-toxic in the amounts produced during use in applications as intended by the present invention.

Another preferred method of manufacturing a carbamate **CA** suitable to be used in a chemical blowing agent as described before comprises the reaction of an isocyanate according to formula (III) with n equivalents of a tertiary alcohol HO-C(R¹)(R²)(R³),

A ⁅NCO]ₙ (III)

wherein the radicals R¹, R², R³ and A and the index n have the same meaning as described before.

Carbamates **CA** manufactured in this way can be synthesized with a large variety of different alcohols and isocyanates according to formula (III). This method thus has the advantage of leading to more tailored products which can be adjusted to the respective application. If using tert-butanol as tertiary alcohol, perferred carbamates **CA** according to the formula (Ia) are obtained.

The reaction of tertiary alcohols HO-C(R¹)(R²)(R³) with isocyanates according to formula (III) to yield carbamates **CA** according to formula (I) is performed following well-known synthetic procedures in polyurethane chemistry. For example, by using a molar excess of tertiary alcohol with regard to the isocyanate (III) and carrying out the conversion in the presence of a dibutyl tin dilaurate (DBTDL) catalyst at 70 °C.

Suitable isocyanates according to formula (III) include linear or branched mono- or polyisocyanates having 1 to 20 carbon atoms which optionally contain cycloaliphatic and/or aromatic moieties. Such isocyanates can have 1 to 4 isocyanate groups, while polyisocyanates with 2 or 3 isocyanate groups are preferred.

Suitable monoisocyanates include methylisocyanate, ethyl isocyanate, n-propylisocyanate, isopropylisocyanate, n-butylisocyanate, isobutylisocyanate, n-hexylisocyanate, isohexylisocyanate, cyclohexylisocyanate and benzylisocyanate.

Preferred suitable polyisocyanates include 1,4-tetramethylene diisocyanate, 2-methylpentamethylene-1,5-diisocyanate, 1,6-hexanediisocyanate (HDI), 2,2,4- and 2,4,4-trimethyl-1,6-hexanediisocyanate (TMDI), 1,10-decanediisocyanate, 1,12-dodecanediisocyanate, lysine or lysine ester diisocyanate, cyclohexane-1,3- or -1,4-diisocyanate, 1-methyl-2,4- and - 2,6-diisocyanatocyclohexane (H₆TDI), 1-isocyanato-3,3,5-trimethyl-5-isocyanatomethylcyclohexane (isophoronediisocyanate or IPDI), perhydro-2,4'- and -4,4'-diphenylmethane diisocyanate (H₁₂MDI), 1,3- or 1,4-bis-(isocyanatomethyl)cyclohexane, m- and p-xylylene diisocyanate (m- and p-XDI), m- and p-tetramethyl-1,4-xylylene diisocyanate (m- and p-TMXDI), bis-(1-isocyanato-1-methylethyl)naphthalene, and dimer fatty acid isocyanates such as 3,6-bis-(9-isocyanatononyl)-4,5-di-(1-heptenyl)cyclohexene (dimeryl diisocyanate).

Especially preferred isocyanates are HDI, TMDI, IPDI and H₁₂MDI. Most preferred isocyanates are HDI and IPDI.

Suitable tertiary alcohols HO-C(R¹)(R²)(R³) include tert-butanol, 2-methyl-2-hexanol, 2,2-dimethyl-3-ethyl-3-pentanol, 2,6-dimethyl-2-heptanol, 3,7-dimethyl-3-octanol, 4-hydroxy-4-methyl-2-pentanone, 1-methylcyclohexanol, 1-methylcyclopentanol, 2-ethylfenchol, 1,1-diphenylethanol, 1,2-diphenyl-2-propanol, 2-methyl-2-butanol, 2-phenyl-2-propanol, 2-phenyl-2-butanol, 2-methyl-1-phenyl-2-propanol, 2,3-dimethyl-2-butanol, 2,3-dimethyl-3-pentanol, 3-methyl-3-pentanol, 3-ethyl-3-pentanol, 2-methyl-4-phenyl-2-butanol, 2-methyl-3-butyne-2-ol, 2-methyl-3-butene-2-ol (dimethylvinylcarbinol), 1,1-dimethoxy-2-methyl-3-butene-2-ol, 3,7-dimethyl-1,6-octadiene-3-ol (linalool), 3,7-dimethyl-6-octene-3-ol (dihydrolinalool), 3,7-dimethyl-3-octanol (tetrahydrolinalool), 3-methyl-1-pentyne-3-ol, 3-methyl-1-pentene-3-ol, and 3,5-dimethyl-1-hexyne-3-ol, as well as hydrogenated derivates of those mentioned with unsaturated C-C bonds. The most preferred tertiary alcohol HO-C(R¹)(R²)(R³) is *tert*-butanol.

Other preferred embodiments use tertiary polyols, i.e. molecules with more than one tertiary alcohol group HO-C(R¹)(R²)(R³), in particular diols with two tertiary alcohol groups. These polyols may be used in the same way as the tertiary alcohols mentioned above in order to form carbamates **CA** according to formula (I). However, when reacting them with mono- or polyisocyanates, care has to be taken to correctly calculate the molar ratio of hydroxyl groups to isocyanate groups. The calculation of a correct equivalent ratio of these functional groups is well known to the ordinarily skilled person in the art. In the case of tertiary polyols, it may be advantageous to use small (low molecular weight) and/or monofunctional isocyanates as reaction partners for the tertiary polyols, in order to achieve a high functional density of carbamate functions on the thus formed carbamate **CA**. Suitable tertiary polyols include 2,3-dimethyl-2,3-butanediol, 3,6-dimethyloct-4-yne-3,6-diol, 2,4,7,9-tetramethyldec-5-yne-4,7-diol, and larger ones, such as 2,6,9,13-tetramethyltetradeca-2,12-dien-7-yne-6,9-diol, and hydrogenated variants thereof. Most preferred tertiary polyols are 2,3-dimethyl-2,3-butanediol and . 2,4,7,9-tetramethyldec-5-yne-4,7-diol.

In preferred embodiments, compositions comprising the chemical blowing agent according to formula (I) furthermore comprise at least one activator **AC** for decomposing the at least one carbamate **CA.**

Although it is possible that any embodiment of carbamate **CA** may decompose in a suitable fashion at a suitable temperature for a certain application without the use of an activator **AC,** it is generally preferred to use an activator **AC**. Such an activator catalyzes or facilitates the decomposition of carbamate **CA** in a way that lower decomposition temperatures and/or a faster decomposition can be achieved, which may be beneficial for the expansion process. The decomposition temperature of a given carbamate **CA** depends largely on its chemical structure, a fact which is discussed further below.

Suitable activators **AC** are all compounds which catalyze or activate the decomposition of carbamate **CA**. Depending on the respective structure of carbamate **CA**, such compounds may include organic or inorganic acids, bases, or metal complexes.

In preferred embodiments, activator **AC** comprises at least one acid, preferably a carboxylic acid. Suitable acids include in particular carboxylic acids which are solid at room temperature (23 °C). Both monoacids and polyacids are suitable.

In an especially preferred embodiment, activator **AC** comprises at least one carboxylic acid with a pKa value of between 2.5 and 5. It is possible to use stronger carboxylic acids, such as trifluoroacetic acid, which are even more efficient in decomposing carbamates **CA**. However, it is preferred to use weaker carboxylic acids with pKa values of between 2.5 and 5 in order to maintain a useful stability of the carbamate **CA**. By using stronger acids, it is possible that carbamate **CA** decomposes too easily, such as at room temperature, and the blowing agent may not be stable enough to be used in a product with acceptable shelf life. It is important to adjust carbamate **CA** and activator **AC** to the desired application. Generally it is favorable that carbamate **CA** does not decompose significantly below a temperature of between 90 °C and 150 °C, but this depends on the intended application.

Preferred activators **AC** include carboxylic acids, in particular those which are solid at room temperature (23°C) and/or exhibit a pKa value of between 2.5 and 5. Examples of such preferred activators **AC** include monocarboxylic acids such as C10 to C20 fatty acids, aromatic carboxylic acids such as benzoic acid or salicylic acid, or polycarboxylic acids such as adipic acid, fumaric acid, or citric acid. Most preferred is salicylic acid.

In preferred embodiments where carboxylic acids are used in activator **AC,** the chemical blowing agent exhibits a molar ratio of carbamate functions of said carbamate **CA** to carboxylic acid functions of said activator **AC** of approximately 1:1.

The heat required for the endothermic decomposition reaction of carbamate **CA**, which causes the foaming (expansion) of a composition containing a blowing agent comprising said carbamate **CA**, can be applied externally or internally, the latter e.g. from an exothermic reaction. The exact decomposition temperature depends largely on the structure of the carbamate **CA** and whether an activator **AC** is used and if so, the kind of activator **AC**. Without using an activator **AC,** the decomposition temperature of carbamates **CA** are normally found approximately between 200 °C and 270 °C. By using a suitable activator **AC,** the decomposition temperature can be lowered significantly, for instance approximately to between 150 °C and 220 °C, depending on the carbamate **CA** and activator **AC** used.

Another aspect of the present invention is the use of a chemical blowing agent comprising at least one carbamate **CA**, as described above, preferably in combination with an activator **AC,** as described above, as a blowing agent in thermally expandable thermoplastic compositions.

The inventive thermally expandable composition preferably contains said chemical blowing agent (comprising or essentially consisting of carbamate **CA**) in an amount of between 2 wt.-% and 10 wt.-%, preferably between 4 wt.-% and 8 wt.-%, more preferably between 5 wt.-% and 7 wt.-%, based on the total weight of the composition.

Such a thermally expandable composition contains, apart from the chemical blowing agent which was described further above, at least one thermoplastic material which can be foamed under controllable conditions by decomposing said chemical blowing agent contained therein.

In a preferred embodiment the thermoplastic material is a curable thermoplastic material. The term "curable thermoplastic material" denotes a thermoplastic material which softens at a certain temperature but is essentially solid at room temperature (23 °C) and preferably also at slightly elevated temperatures, e.g. up to 50°C or 60°C, and which is preferably able to "cure" or in other words undergo cross-linking reactions under certain conditions, e.g. at elevated temperatures. The use of curable thermoplastic materials affords optimal mechanical properties after expansion and ensures an increased stability of the foam.

The chemistry of the cross-linking reactions depends on the kind of polymers used in the thermoplastic material, on functional groups they carry, and on the presence of further reaction partners such as crosslinkers, initiators or catalysts. The cross-linking reactions may include condensation or addition reactions with ionic or radical mechanisms.

For example, in one preferred embodiment the curable thermoplastic material comprises at least one polymer cross-linkable by peroxides.

Polymers are denoted as "cross-linkable by peroxide" if these polymers contain functional groups, e.g. C-C double bonds, which release hydrogen atoms under influence of a radical starter, e.g. a peroxide, from their backbone or side chain, such that a radical remains that is able to radically attack other polymer chains in a subsequent step, leading to a radical chain reaction cross-linking process and ultimately to a polymer network.

Suitable polymers of this type include, for example, styrene-butadiene copolymers, styrene-isoprene copolymers, ethylene-vinyl acetate copolymers, ethylene-methacrylate copolymers, ethylene-ethyl acrylate copolymers, ethylene-butyl acrylate copolymers, ethylene-(meth)acrylic acid copolymers, ethylene-2-ethylhexyl acrylate copolymers, ethylene-acrylic ester copolymers, polyolefinc block copolymers, and polyolefins such as polyethylene or polypropylene.

The copolymers, meaning polymers made from more than one type of monomer, can be block-type copolymers, regularly alternating copolymers, or random copolymers.

These polymers can also be further functionalised, meaning they can contain further functional groups such as hydroxyl, carboxy, anhydride, acrylate, and/or glycidylmethacrylate groups.

Preferred polymers cross-linkable by peroxide comprise or essentially consist of ethylene-vinyl acetate (EVA). In this case, the content of vinyl acetate monomers in EVA should be between 8 wt.-% and 45 wt.-%, preferably between 15 wt.-% and 30 wt.-%, based on the total weight of the EVA polymer.

Preferred EVA polymers include, e.g., Elvax® 150, Elvax® 240, Elvax® 260, Elvax® 420, Elvax® 670 (all by DuPont), or the corresponding Evatane® copolymers (by Arkema).

Other polymer systems suitable to be comprised in the curable thermoplastic material include for example one-component epoxy resin systems which are essentially solid at room temperature (23 °C) and which exhibit high impact strength or toughness and preferably contain thixotropy modification additives such as pyrogenic silica or nanoclays.

Further suitable epoxy-based thermoplastic materials include crystalline polyepoxides such as triglycidyl isocyanurate, terephthalic acid triglycidyl ether, mixtures of terephthalic acid triglycidyl ether with trimellitic acid triglycidyl ether, hydroquinone diglycidyl ether, as well as adducts of trimethylolpropane diglycidyl ether with diisocyanates such as 4,4'-, 2,4'- and 2,2'-diphenylmethane diisocyanate (MDI), 2,4- and 2,6-toluylene diisocyanate (TDI), or 1-isocyanato-3,3,5-trimethyl-5-isocyanatomethyl-cyclohexane (IPDI).

Especially suitable toughness or impact strength modifiers for these thermoplastic materials include reactive liquid rubbers based on nitrile rubber or derivates of polyetherpolyol polyurethanes, core-shell polymers, and other such systems known to the person skilled in the art.

Other suitable polymer systems for the curable thermoplastic material include one-component polyurethane compositions comprising crystalline, hydroxy-functional polyester or polycarbonate polyols mixed with other polyols, preferably polyether polyols, and polyisocyanates with blocked isocyanate groups. The melting point of the crystalline polyester or polycarbonate polyol should preferably be at least 50 °C. The isocyanate groups of the polyisocyanate can for example be blocked by nucleophiles such as caprolactam, phenols, or benzoxazolones. Furthermore suitable are blocked polyisocyanates as used for instance in powder coating applications and which are commercially available, e.g., under the trade names Vestagon® BF 1350 and Vestagon® BF 1540 (by Evonik). Suitable as polyisocyanates are furthermore so-called encapsulated or surface-deactivated polyisocyanates, which are known to the person skilled in the art and for example described in EP 0 204 970.

Furthermore suitable as curable thermoplastic materials are two-component epoxy/polyurethane compositions which are for example described in WO 2005/080524 A1.

Also suitable as curable thermoplastic materials are those compositions which are for example commercially available under the trade names SikaBaffle® 240, SikaBaffle® 250, or SikaBaffle® 255 (by Sika Corp., USA), and which are described in detail in US 5,266,133 and US 5,373,027. Such curable thermoplastic materials are particularly preferred for the present invention.

Especially suitable as curable thermoplastic material with reinforcement properties, for example in automotive manufacturing, are for example those sold under the trade name SikaReinforcer® 941 (by Sika Corp., USA). Such compositions are described in US 6,387,470.

The thermally expandable composition according to the present invention preferably contains said at least one thermoplastic material in an amount of between 60 wt.-% and 80 wt.-%, preferably between 65 wt.-% and 78 wt.-%, more preferably between 70 wt.-% and 75 wt.-%, based on the total weight of the thermally expandable composition.

The thermally expandable composition according to the present invention preferably comprises at least one stabilizer **S.**

Said stabilizer **S** stabilizes the foam created by the gas of decomposing carbamate **CA** in the thermoplastic material.

Foam stabilization is usually achieved either by means of foam stabilizers and/or by fast crosslinking of the thermoplastic melt. Foam stabilizers help maintaining the stability of the gas bubbles of the foam, which protects the foam from collapsing until the thermoplastic material is cured or solidified enough to maintain the cellular structure.

Suitable foam stabilizers **S** include for example low molecular weight, amphiphilic molecules, such as polyether-modified polysiloxanes (e.g. Tegostab® products by Evonik, or Silbyk® 9000, Silbyk® 9020, and Silbyk® 9230 by Altana Group) or monoglycerides (e.g. glycerol monostearate such as Dimodan HP®, by Danisco). Furthermore suitable is sodium sec-alkanesulfonate as sold under the trade name Armostat® 3002 by AkzoNobel. Other suitable foam stabilizers **S** include siloxane-glycol random copolymers, or long chain branched polymers as for example described in WO 2014/040913 A1. Also suitable stabilizers **S** are thixotropic agents such as calcium carbonate as e.g. sold under the trade name Omyacarb® 5-GU or fumed silica, such as Aerosil® R972 by Evonik or HDK® H18 by Wacker.

In a preferred embodiment of the thermally expandable composition according to the present invention, said stabilizer **S** is contained in an amount of between 0.1 wt.-% and 3 % wt.-%, preferably between 0.2 wt.-% and 2 wt.-%, more preferably between 0.25 wt.-% and 1 wt.-%, based on the total weight of the thermally expandable composition.

In preferred embodiments, said inventive thermally expandable composition furthermore comprises an activator **AC** for said carbamate **CA.** The activator **AC** was described in detail further above. In especially preferred embodiments said activator **AC** comprises at least one carboxylic acid, preferably a carboxylic acid with a pKa value of between 2.5 and 5. Furthermore in especially preferred embodiments of such expandable compositions according to the present invention described above, the molar ratio of carbamate functions of said carbamate **CA** to carboxylic acid functions of said activator **AC** is 1:1 or approximately 1:1.

Apart from the essential and optional components such as summarized under a), b), and c) further above, the present inventive thermally expandable composition may contain other components commonly used in such compositions and known to the person skilled in the art. These include, for example, fillers, colorants, dispersion aids or homogenizers, adhesion promoters, antioxidants, stabilizers, and the like.

Suitable as fillers are, e.g., ground or precipitated calcium carbonate, calcium-magnesium carbonate, talcum, gypsum, graphite, barite, silica, silicates, mica, wollastonite, carbon black, or the mixtures thereof, or the like. Fillers are, if at all, preferably contained in an amount of between 1 wt.-% and 15 wt.-%, based on the total weight of the composition.

Colorants or dyes, such as pigments, e.g. on the basis of carbon black, may be contained in the present inventive compositions. Their amount is preferably between 0 wt.-% and 1 wt.-%, based on the total weight of the composition.

Dispersion aids or homogenizers, sometimes described as wetting agents or surface-active agents, may be beneficial for the present inventive composition in order to facilitate a homogeneously mixed composition. Preferably used such compounds include hydrocarbon resins, for example Novares® TL 90 (by Rütgers), Wingtack® resins (by Cray Valley), Escorez® tackifying resins (e.g., Escorez® 1304, by ExxonMobil), and Piccotac® hydrocarbon resins (e.g., Piccotac® 1100 or Piccotac® 1100E, by Eastman). Such compounds are preferably contained in an amount of between 2 wt.-% and 10 wt.-%, preferably between 4 wt.-% and 8 wt.-%, more preferably between 5 wt.-% and 7 wt.-%, based on the total weight of the composition.

In preferred embodiments, the inventive composition also comprises adhesion promoters. Preferably these substances are incorporated into the polymer network during the cross-linking reactions via functional groups similar to those present the curable thermoplastic material, where applicable. Suitable adhesion promoters include, for example, ethylene-glycidyl methacrylate copolymers, such as Lotader® ADX 1200S, Lotader® AX8840, Lotader® 3210, Lotader® 3410 (by Arkema) or Lotryl® copolymers (by Arkema).

Adhesion promoters are preferably contained in an amount of between 2 wt.-% and 15 wt.-%, preferably between between 4 wt.-% and 10 wt.-%, more preferably between 5 wt.-% and 7 wt.-%, based on the total weight of the composition.

Further potentially useful additives include antioxidants and stabilizers, commonly used in polymer-based compositions and known to the person skilled in the art of polymer-based composition formulation. Examples of suitable antioxidants and stabilizers include sterically hindered thioethers, sterically hindered aromatic amines, and/or sterically hindered phenols, such as bis(3,3-bis(4'-hydroxy-3-t-butylphenyl)butanoic acid) glycol ester and pentaerythritol tetrakis(3-(3,5-di-*tert*-butyl-4-hydroxyphenyl)propionate), known under the trade name Irganox® 1010 (by BASF). Such substances are preferably contained in an amount of between 0 wt.-% and 0.5 wt.-%, preferably between 0.1 wt.-% and 0.3 wt.-%, based on the total weight of the composition.

The compositions according to the present invention can be manufactured by mixing the components in any suitable mixing apparatus, e.g. in a dispersion mixer, planetary mixer, double screw mixer, continuous mixer, extruder, or twin screw extruder.

It may be advantageous to heat the components before or during mixing, either by applying external heat sources or by friction generated by the mixing process itself, in order to facilitate processing of the components into a homogeneous mixture by decreasing viscosities and/or melting of individual components. However, care has to be taken, e.g. by temperature monitoring and use of cooling devices where appropriate, not to exceed the activation temperatures of the blowing agent. The final composition is preferably essentially solid at room temperature (23 °C), meaning that it does not visibly deform at this temperature just by means of gravity during at least 24 h.

After mixing, the resulting composition may be shaped into its desired form by, e.g., extruding, blow-moulding, pelleting, injection moulding, compression moulding, punching or stamping or any other suitable process.

The thermally expandable compositions may be produced in a substantially one-step process, involving the addition of all components in a series and/or simultaneously. However, it may also be advantageous to formulate the composition as a two-part system, or even multi-part system, and mix these parts into the final composition at a later stage. Such an approach may, for example, increase shelf life of the composition in places with demanding conditions (such as extraordinarily high temperatures), optimise storage room demand and transport weight, and allow for tailor-made, modular compositions regarding different applications.

The expansion of the thermally expandable composition according to the present invention is normally triggered by heat. This means that both the blowing agent comprising carbamate **CA** and in preferred cases the curing mechanism of the curable thermoplastic material are activated by a thermal process which exceeds their respective activation temperature and exhibits a duration long enough for both processes (curing process such as for example peroxide-initiated radical polymerisation and decomposition of the blowing agent including gas formation) to proceed until the expandable material has expanded and cured into its intended final (sufficiently expanded and stable) state. The optimal temperature and duration (dwell time) depends on the blowing agent and the properties of the thermoplastic material used in the inventive composition. Commonly, such activation temperatures are in the range of 130 °C to 250 °C, preferably 150 °C to 210 °C, and require a dwell time of between 10 min and 90 min, preferably between 15 min and 60 min.

Another aspect of the present invention is a baffle and/or reinforcement element for hollow structures, wherein said element comprises a thermally expandable composition as described above and optionally a carrier on which said expandable material is deposited or attached.

In more detail, this aspect of the present invention also describes the use of inventive thermally expandable compositions as described above containing the chemical blowing agent for the manufacturing of baffle and/or reinforcement elements. Such elements are used to seal, baffle, and/or reinforce hollow structures, e.g. a cavity in a hollow structural part of an automobile. Hollow parts in cars may include body components (e.g., panels), frame components (e.g., hydroformed tubes), pillar structures (e.g., A, B, C, or D pillars), bumpers, roofs, or the like.

In one preferred embodiment, such a baffle and/or reinforcement element for hollow structures consists essentially of a thermally expandable composition as described above. In this case, it is advantageous to design the shape of the element in a way that it can be easily fitted into and attached to the walls of the hollow structure to be baffled and/or reinforced. Manufacturing is in this case preferably done by injection moulding, compression moulding, punching or stamping, or extrusion through a shape template.

In another preferred embodiment, such a baffle and/or reinforcement element for hollow structures comprises, apart from the thermally expandable composition, a carrier element on which the inventive thermally expandable composition is deposited or attached. Such a design may be more cost-efficient and it may facilitate fixation of the baffle and/or reinforcement element on the walls of the structure to be baffled and/or reinforced, e.g. by incorporation of pins, bolts, or hooks on the carrier element. Furthermore, with a suitable design of the carrier element, the mechanical performance and stability of the baffle and/or reinforcement element according to the present invention can be increased.

Said carrier element may consist of any material that can be processed into a shape useable for an embodiment of the present invention. Preferred materials are polymeric materials, such as elastomers, thermoplastics, thermosetting polymers, a blend or other combination thereof, or the like. Preferred thermoplastic materials include, without limitation, polymers such as polyurethanes, polyamides, polyesters, polyolefins, polysulfones, poly(ethylene terephthalates), polyvinylchlorides, chlorinated polyolefins, or the like. Especially preferred are high-temperature stable polymers such as poly(phenylene ethers), polysulfones, polyethersulfones, polyamides, preferably PA 6, PA 6,6, PA 11, PA 12, PA 6,10, PA 6,12, or a mixture thereof. Other suitable materials include metals, especially aluminium or steel, or naturally grown, organic materials, such as wood or other (pressed) fibrous materials. Also glassy or ceramic materials can be used. It is possible to use any combination of such materials. Such materials can also be filled (e.g. with fibres, minerals, clays, silicates, carbonates, combinations thereof or the like) or foamed.

The carrier element can further exhibit any shape or geometry. It can also consist of several, not directly connected parts. For example, it can be massive, hollow, or foamed, or it can exhibit a grid-like structure. The surface of the carrier element can typically be smooth, rough, or structured, according to the intended use of the baffle and/or reinforcement element.

The manufacturing process of a baffle and/or reinforcement element in accordance with the present invention depends largely on the material of the carrier element. If the material of the carrier element can be processed by injection moulding or extrusion, the whole baffle and/or reinforcement element can be produced in a two-step injection moulding process or a co-extrusion process of the carrier element and the thermally expandable composition. If using a two-step injection moulding process, in a first step, material for the carrier element is injected into the mould. After solidification, the cavity of the injection moulding tool is enlarged or adjusted, or the injection-moulded piece is transferred into another tool and the second component, in this case the material for the thermally expandable composition, is injected.

If the carrier element is not shaped by injection moulding or extrusion, e.g., because it consists of a metal or alloy, it may be first manufactured by a suitable process and then introduced into the injection moulding tool, and the thermally expandable composition may be injection-moulded into the tool where the carrier element was placed. Another possibility is to extrude the thermally expandable composition onto the pre-fabricated carrier element. Of course there is also the possibility of manufacturing the carrier element and the expandable composition element individually by a suitable process, and then attaching the expandable composition element to the carrier element by any suitable means, such as chemically or physically, e.g. by gluing or the like, or mechanically, e.g. by bolting, screwing, or the like.

Another aspect of the present invention is the use of the baffle and/or reinforcement element as described above to seal, baffle, or reinforce a cavity or hollow structure of a land vehicle, watercraft, or aircraft, preferably an automobile, and/or a cavity of a building such that the transmission of noise, vibrations, humidity, and/or heat is reduced, and/or the object surrounding said cavity is mechanically strengthened.

A further aspect of the present invention is a method for sealing, baffling and/or reinforcing a cavity or hollow structure, characterised in that an element comprising the thermally expandable composition as described above is introduced into said cavity or hollow structure and subsequently thermally expanded such that said cavity or hollow structure is at least partially filled by the expanded composition. Preferred temperature for the thermal expansion process is between 130 °C and 210 °C.

If the thermally expandable composition as described above finds use in a baffle and/or reinforcement element, e.g. in automotive manufacturing, it is preferable that the activation temperature of the blowing agent is adjusted to the manufacturing conditions of the automotive part to be baffled or reinforced. As an example, the baffle and/or reinforcement element can be inserted into a cavity of a structure that needs to be treated by an electro-coating solution, in its unexpanded state still leaving the surface of the structure accessible, and subsequently, during the heat treatment of the automotive part (i.e. the curing procedure for the cathodic dip painting/coating or electro-coating solution), the baffle and/or reinforcement element simultaneously (or shortly thereafter) expands to its intended final shape and at least partially closes or fills the cavity. In such a case, the expansion temperature should correspond to the temperature conditions of said heat treatment, i.e. to between 90 °C and 200 °C.

The invention is further explained in the following experimental part which, however, shall not be construed as limiting the scope of the invention.

### Examples

The proportions and percentages indicated are by weight, unless otherwise stated. Accordingly, "wt.-%" means percentage by weight, based on the weight of the total composition given in the respective case.

### Test methods and procedures

**¹H- and ¹³C-NMR** spectra were measured in CDCl₃ on a Bruker Ascend 400 spectrometer at 400.14 MHz, the chemical shifts are given in ppm relative to tetramethyl silane (TMS).

**Fourier-transform infrared** (FT-IR) **spectra** were recorded as films or powders in undiluted form on a horizontal attenuated total reflectance (ATR) diamond unit on a Nicolet iS5 spectrometer by Thermo Scientific with a resolution of 1 cm⁻¹. Absorption bands are given in wavenumbers (cm⁻¹) for the measurement range of 4000-650 cm⁻¹.

**Differential scanning calorimetry** (DSC) measurements were performed on a DSC 1 differential scanning calorimeter by Mettler Toledo using the following procedure: 2 min at 25 °C, heating up from 25 °C to 280 °C with 5 °C/min, 2 min at 280 °C, cooling down from 280 °C to 25 °C with 10 °C/min. Decomposition temperature range (°C), decomposition temperature onset (°C), peak decomposition temperature (°C) and enthalpy (J/kg) as well as melting points Tₘ (°C) were measured.

Activator/carbamate mixtures were ran at three different heating rates (10, 20 and 30°C/min.) using the DSC procedure described above. Decomposition peaks of all three runs were evaluated using the automatic kinetic (MFK) procedure of the STARe Software (Mettler Toledo, Switzerland) generating an activation energy plot. The activation energy curve was used to generate conversion (decomposition rate) plots and tables (see Tables 4 and 5 below).

**Thermal gravimetric analysis** (TGA) measurements were performed on a Mettler Toledo TGA 1 machine using the following parameters: Heating up from 30 °C to 250 °C with 5 °C/min, followed by a second heating procedure from 250 °C to 900 °C with 40 °C/min. The recorded values include decomposition temperature range (°C), decomposition onset (°C), peak decomposition temperature (°C), mass loss (%), and rest (residual) mass (%).

**Gas volume** and decomposition temperature by gas evolution equipment were measured at Tramaco GmbH (Germany) using the following procedure: Principle: In a volumetric measuring tube a water column was generated by suction, which exerts a low depression to a sample tube. During the thermal decomposition of the blowing agent, the water column decreases proportional to the volume of gas released until a low depression is formed again which prevents any further decrease of the water column. The water which drops out of the volumetric measuring tube is collected and continuously weighed and the mass recorded synchronously to the temperature. Evaluation: Gas volume (normalized) and decomposition temperature (measured range from 80 °C to 300 °C) were recorded by the equipment software during each measurement (gas volume in mL/g = water weight / initial sample weight * correction factor). The software evaluates the recorded data and displays the gas volume as a function of temperature (x/y diagram). After decomposition is complete, the curve rises no further.

### Synthesis of example compounds

A series of carbamates were synthesized according to the following procedures.

### Synthesis of example carbamates CA1 to CA7 and CA10 to CA18 from amines

A series of example carbamates **CA** were produced by a first method using polyamines and di-*tert*-butyl dicarbonate as starting materials to yield carbamates **CA**. All reactions were performed in THF at 25 °C, with triethylamine as catalyst and using an equimolar amount of di-*tert*-butyl dicarbonate with respect to amino groups. In all cases, the corresponding carbamates could be obtained with between 90 and 99% yield and characterized by ¹H-NMR and ¹³C-NMR and FT-IR spectroscopy. The chemical names of the polyamines used and the corresponding carbamates **CA** obtained are given in Table 1. The reactants (including the amines) were all purchased from Sigma Aldrich, Switzerland, except for the amines used for CA4 and CA5, which were purchased from Huntsman Corp., USA.

**Table 1: Amines used in the synthesis of carbamates CA1 to CA7 and CA10 to CA18, including the chemical names of the products CA and their appearance and melting points (DSC).**

| | Amine | Carbamate **CA** | Properties |
|---|---|---|---|
| CA1 | Dodecane-1,12-diamine | Di-*tert*-butyl dodecane-1,12-diyldicarbamate | Crystalline (white), Tₘ = 109 °C |
| CA2 | 2,2-Dimethylpropane-1,3-diamine | Di-*tert*-butyl 2,2-dimethylpropane-1,3-diyldicarbamate | Crystalline (white) Tₘ = 130 °C |
| CA3 | 4-(Aminomethyl)octane-1,8-diamine | Di-*tert*-butyl 4-(((*tert-*butoxycarbonyl)amino)methyl )octane-1,8-diyldicarbamate | Liquid (opaque) |
| CA4 | 3-Oxapentane-1,5-diamine (Jeffamine® EDR-104) | Di-*tert*-butyl 3-oxapentane-1,5-diyldicarbamate | Crystalline (white) Tₘ = 69 °C |
| CA5 | 4,7-Dioxadecane-1,10-diamine (Jeffamine® EDR-176) | Di-*tert*-butyl 4,7-dioxadecane-1,10-diyldicarbamate | Liquid (opaque) |
| CA6 | Ethane-1,2-diamine | Di-*tert*-butyl ethane-1,2-diyldicarbamate | Crystalline (white) Tₘ = 140 °C |
| CA7 | Propane-1,2-diamine | Di-*tert*-butyl propane-1,2-diyldicarbamate | Crystalline (white) Tₘ = 121 °C |
| CA10 | 1-Butylamine | *tert*-Butyl butylcarbamate | Colorless liquid |
| CA11 | 3-Dimethylaminopropylamine | *tert*-Butyl (3-(dimethylamino)propyl)-carbamate | Yellow liquid |
| CA12 | 1,4-Bis(2-aminoethyl)piperazine | Di-*tert*-butyl (piperazine-1,4-diylbis(ethane-2,1-diyl))dicarbamate | White powder |
| CA13 | Pyrrolidine | *tert*-Butyl pyrrolidine-1-carboxylate | Colorless liquid |
| CA14 | Piperazine | Di-*tert*-butyl piperazine-1,4-dicarboxylate | White powder |
| CA15 | Morpholine | *tert*-Butyl morpholine-4-carboxylate | White crystals |
| CA16 | Dimethylamine | *tert*-Butyl dimethylcarbamate | Yellow liquid |
| CA17 | 2-Aminoethanol | *tert*-Butyl (2-hydroxyethyl)carbamate | Colorless liquid |
| CA18 | 1-Amino-2-propanol | *tert*-Butyl (2-hydroxypropyl)carbamate | Colorless liquid |

### Characterization data of di-tert-butyl ethane-1,2-diyldicarbamate (CA6)

As an example for chemical characterization data by NMR and FT-IR, the spectral assignments of ¹H- and ¹³C-NMR and FT-IR are given for CA6, which is one of the most preferred embodiments of the carbamate **CA**:
¹H-NMR (400 MHz, CDCl₃): δ 4.88 (s, 2H, NH), 3.23 (s, 4H, CH₂), 1.44 (s, 18H, CH₃);
¹³C-NMR (100 MHz, CDCl₃): δ 156.3 (C=O), 79.4 (C), 40.8 (CH₂), 28.0 (CH₃); FT-IR (most important bands): 3374.1 (NH), 2867.7, 2935.1, 2982.5 (CH₃, CH₂), 1680.9 (C=O), 1523.2 (NH), 1461.9, 1367.0 (CH₃, CH₂), 1140.2 (N-C).

### Synthesis of example carbamates CA8, CA9, CA19, and CA20 from isocyanates

Two example carbamates **CA** were produced by a second method using polyisocyanates and alcohols as starting materials to yield carbamates **CA**. Both reactions were performed in tert-butanol which served also as reactant, at 50 °C, with dibutyl tin dilaurate (DBTDL) as catalyst. Furthermore, two additional carbamates were prepared by reacting 1,6-hexanediisocyanate (HDI) with other alcohols than tert-butanol, namely 2-methyl-2-butanol (CA19) and 2,4,7,9-tetramethyl-5-decyne-4,7,diol (CA20). In all cases, the corresponding carbamates could be obtained with between 80 to 95% yield and characterized by ¹H- and ¹³C-NMR and FT-IR spectroscopy. The chemical names of the polyisocyanates used and the corresponding carbamates **CA** obtained are given in Table 2. The reactants were all purchased from Sigma Aldrich, Switzerland.

**Table 2: Isocyanates used in the synthesis of carbamates CA8, CA9, CA19, and CA20 including the chemical names of the products.**

| Example | Isocyanate | Carbamate **CA** |
|---|---|---|
| CA8 | 1,6-Hexanediisocyanate (HDI) | Di-*tert*-butyl hexane-1,6-diyldicarbamate |
| CA9 | Isophoronediisocyanate (IPDI) | *tert*-butyl ((5-((*tert-*butoxycarbonyl)amino)-1,3,3-trimethylcyclohexyl)methyl)carbamate |
| CA19 | 1,6-Hexanediisocyanate (HDI) | Di-*tert*-pentyl hexane-1,6-diyldicarbamate |
| CA20 | 1,6-Hexanediisocyanate (HDI) | C₂₂H₃₈N₂O₄ (repeating unit of oligomer) |

### Gas volume and decomposition temperature

Table 3 shows the decomposition temperature and the gas volume for various chemical blowing agents, including reference compounds and carbamates **CA.**

**Table 3: Decomposition temperature or temperature range and decomposition gas volume of selected reference blowing agents (denoted "Ref") and selected carbamates CA.**

| *Entry* | Substance | Decomposition temperature [°C] | Gas volume [mL/g] |
|---|---|---|---|
| *1 (Ref)* | Azodicarbonamide (ADCA) | 205-215 | 220 |
| *2 (Ref)* | NaHCO₃ | 130-150 | 120 |
| *3 (Ref)* | Citric acid | 180-230 | 120 |
| *4 (Ref)* | Oxybis(benzenesulfonylhydrazide) (OBSH) | 155-165 | 125 |
| *5 (Ref)* | Toluenesulfonylhydrazide (TSH) | 105-110 | 110 |
| *6 (Ref)* | *p*-Toluenesulfonylsemicarbazide (TSSC) | 228-235 | 120 |
| *7 (Ref)* | 5-Phenyltetrazole (5-PT) | 215-225 | 175 |
| *8 (Ref)* | *N*,*N'*-Dinitrosopentamethylene tetramine (DNPT) | 200 | 220 |
| *9* | CA1 | 213 | 150 |
| *10* | CA2 | 202 | 165 |
| *11* | CA4 | 192 | 200 |
| *12* | CA5 | 212 | 145 |
| *13* | CA6 | 185 | 205 |

The carbamates generally produce higher gas volume than the reference compounds, apart from ADCA (entry 1), DNPT (entry 8) and, to a lesser extent, 5-PT (entry 7). DNPT has the disadvantage of producing toxic carbon monoxide (CO) when decomposing.

### Influence of activator

To show the influence of an activator **AC** on the blowing agent comprising a carbamate **CA**, Tables 4 and 5 show the time (in minutes) required for decomposing carbamate CA6 at various temperatures (decomposition rate). Table 4 shows the decomposition rate at different temperatures, i.e. the time to reach selected stages of decomposition (between 5% and 95%) without activator, while Table 5 shows the same experiment but including salicylic acid as activator. In both cases the amount of carbamate CA6 was the same. In the second experiment (Table 5), the molar ratio of carbamate **CA** to activator **AC** was 1:1.

**Table 4: Decomposition rates of carbamate CA6 without activator.**

| Decomposition | 150 °C [min] | 170 °C [min] | 190 °C [min] | 200 °C [min] | 210 °C [min] | 230 °C [min] |
|---|---|---|---|---|---|---|
| 5% | 34.7 | 11.1 | 3.9 | 2.4 | 1.5 | 0.8 |
| 10% | - | 25.6 | 7.5 | 4.2 | 2.4 | 0.9 |
| 30% | - | - | 31.8 | 15.0 | 7.3 | 1.9 |
| 50% | - | - | 74.2 | 32.4 | 14.5 | 3.3 |
| 70% | - | - | - | 61.4 | 26.2 | 5.3 |
| 90% | - | - | - | - | 51.2 | 9.6 |
| 95% | - | - | - | - | 51.4 | 13.8 |

Without activator, carbamate CA6 decomposes efficiently only at temperatures well above 200 °C. Even at 230 °C, the time required to decompose 95% of the material is almost 14 min.

**Table 5: Decomposition rates of carbamate CA6 with activator salicylic acid.**

| Decom-position | 150 °C [min] | 170 °C [min] | 190 °C [min] | 200 °C [min] | 210 °C [min] | 230 °C [min] |
|---|---|---|---|---|---|---|
| 5% | 3.2 | 0.3 | <0.1 | <0.1 | <0.1 | <0.1 |
| 10% | 6.5 | 0.9 | 0.1 | <0.1 | <0.1 | <0.1 |
| 30% | 19.9 | 3.9 | 0.9 | 0.5 | 0.2 | <0.1 |
| 50% | 38.5 | 8.1 | 2.0 | 1.0 | 0.5 | 0.2 |
| 70% | - | 13.3 | 3.3 | 1.7 | 0.9 | 0.3 |
| 90% | - | 18.9 | 5.3 | 2.9 | 1.6 | 0.6 |
| 95% | - | 20.5 | 6.2 | 3.5 | 2.0 | 0.7 |

Using salicylic acid as activator in combination with carbamate CA6, the decomposition proceeds much faster. Already at temperatures below 200 °C useful decomposition rates are achieved.

### Influence of molecular weight

Table 6 lists decomposition peak temperatures and molecular weights of example carbamates **CA**. It is apparent from the data presented that the molecular weight has an influence on the decomposition behavior of carbamates according to formula (I). The higher the molecular weight of a carbamate **CA**, the higher is its decomposition peak temperature. This shows that carbamates **CA** according to formula (I) can be tailored to a specific application by modifying their chemical structure, such as for example their molecular weight.

**Table 6: Decomposition peak temperatures versus molecular weights for various carbamates CA (without activator).**

| Entry | Carbamate CA | Molecular weight [g/mol] | Decomposition peak temperature [°C] |
|---|---|---|---|
| 1 | CA6 | 262.3 | 205 |
| 2 | CA2 | 302.4 | 227 |
| 3 | CA4 | 304.4 | 236 |
| 4 | CA5 | 376.5 | 244 |
| 5 | CA1 | 400.6 | 259 |
| 6 | CA3 | 473.7 | 269 |

**Table 7: TGA data of selected carbamates CA.**

| Entry | Carbamate CA | Mass loss (%) | Decomposition onset (°C) | Peak decomposition temperature (°C) |
|---|---|---|---|---|
| 1 | CA8 | 80.8 | 184 | 205 |
| 2 | CA10 | 100 | 115 | 136 |
| 3 | CA11 | 99 | 138 | 161 |
| 4 | CA12 | 69 | 220 | 240 |
| 5 | CA13 | 100 | 104 | 127 |
| 6 | CA14 | 99.4 | 172 | 199 |
| 7 | CA15 | 99.5 | 121 | 146 |
| 8 | CA16 | 98 | 77 | 102 |
| 9 | CA17 | 99 | 159 | 183 |
| 10 | CA18 | 99 | 156 | 184 |
| 11 | CA19 | 76.6 | 183 | 204 |
| 12 | CA20 | 62.5 | 194 | 209 |

## Claims

1. Thermally expandable composition, comprising
a) at least one chemical blowing agent,
b) at least one thermoplastic material,
c) optionally at least one stabilizer **S**,
wherein
the at least one chemical blowing agent comprises at least one carbamate **CA** according to formula (I), 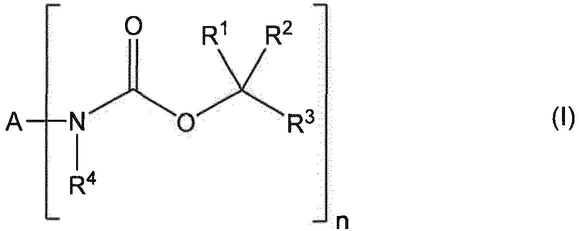 wherein
R¹ and R² independently represent linear or branched monovalent hydrocarbon radicals having 1 to 10 carbon atoms, preferably 1 to 5 carbon atoms, which optionally contain one or more heteroatoms, and/or C-C multiple bonds, and/or cycloaliphatic, and/or aromatic moieties, or together represent a divalent hydrocarbon radical having 4 to 10 carbon atoms, preferably 4 to 6 carbon atoms;
R³ represents a linear or branched monovalent hydrocarbon radical having 1 to 20 carbon atoms, preferably 1 to 10 carbon atoms, and optionally contains one or more heteroatoms, in particular one or more optionally substituted tertiary alkyl carbamates, and/or C-C multiple bonds, and/or cycloaliphatic, and/or aromatic moieties;
R⁴ represents a hydrogen radical or a linear or branched monovalent hydrocarbon radical having 1 to 10 carbon atoms, preferably 1 to 5 carbon atoms, which optionally contain one or more heteroatoms, and/or C-C multiple bonds, and/or cycloaliphatic, and/or aromatic moieties, or together with A represents an (n+1)-valent hydrocarbon radical having 4 to 40 carbon atoms, preferably 4 to 20 carbon atoms, which optionally contains one or more heteroatoms, and/or cycloaliphatic moieties;
A represents a linear or branched n-valent hydrocarbon radical having 1 to 20 carbon atoms, which optionally contains one or more heteroatoms, in particular in the form of ether oxygen or amine nitrogen, and optionally one or more C-C multiple bonds, and/or cycloaliphatic, and/or aromatic moieties, or together with R⁴ represents an (n+1)-valent hydrocarbon radical having 4 to 40 carbon atoms, preferably 4 to 20 carbon atoms, which optionally contains one or more heteroatoms, and/or cycloaliphatic moieties;
index n stands for a value of 1 or 2 or 3 or 4, preferably 2 or 3.

2. Thermally expandable composition according to claim 1, wherein said index n equals 2 and said radical A represents a linear or branched divalent hydrocarbon radical having 2 to 10 carbon atoms and which optionally contains one or more heteroatoms, in particular in the form of ether oxygen.

3. Thermally expandable composition according to claim 1 or 2, wherein said carbamate **CA** has a molecular weight of between 131 g/mol and 750 g/mol, preferably between 131 g/mol and 500 g/mol, more preferably between 131 g/mol and 450 g/mol.

4. Thermally expandable composition according to any of claims 1 to 3, wherein at least 50% of said carbamate **CA** decomposes under gas release at a temperature of between 170 °C and 230 °C within 10 min.

5. Thermally expandable composition according to any of claims 1 to 4, wherein said radicals R¹, R², and R³ represent methyl groups.

6. Thermally expandable composition according to any of claims 1 to 5, wherein said stabilizer **S** is contained in an amount of between 0.1 wt.-% and 3 % wt.-%, preferably between 0.2 wt.-% and 2 wt.-%, more preferably between 0.25 wt.-% and 1 wt.-%, based on the total weight of the thermally expandable composition.

7. Thermally expandable composition according to any of claims 1 to 6, furthermore comprising an activator **AC** for said carbamate **CA**, wherein said activator **AC** comprises at least one carboxylic acid, preferably a carboxylic acid with a pKa value of between 2.5 and 5, in particular salicylic acid.

8. Thermally expandable composition according to claim 7, wherein the molar ratio of carbamate functions of said carbamate **CA** to carboxylic acid functions of said activator **AC** is 1:1.

9. Baffle and/or reinforcement element for hollow structures, wherein said element comprises a thermally expandable composition according to any of claims 1 to 8 and optionally a carrier on which said expandable composition is deposited or attached.

10. Use of a carbamate **CA** according to claim 1, preferably in combination with an activator **AC**, as blowing agent in thermally expandable compositions.

11. Use of the baffle and/or reinforcement element of claim 9 to seal, baffle, or reinforce a cavity or hollow structure of a land vehicle, watercraft, or aircraft, preferably an automobile, and/or a cavity of a building such that the transmission of noise, vibrations, humidity, and/or heat is reduced, and/or the object surrounding said cavity is mechanically strengthened.

12. Method for sealing, baffling and/or reinforcing a cavity or hollow structure, **characterized in that** an element comprising a thermally expandable composition according to any of claims 1 to 8 is introduced into said cavity or hollow structure and subsequently thermally expanded such that said cavity or hollow structure is at least partially filled by the expanded composition.

## Patentansprüche

1. Wärmedehnbare Zusammensetzung, umfassend
a) mindestens ein chemisches Treibmittel,
b) mindestens ein thermoplastisches Material,
c) wahlweise mindestens einen Stabilisator **S**,
wobei
das mindestens eine chemische Treibmittel mindestens ein Carbamat **CA** der Formel (I) entsprechend umfasst, 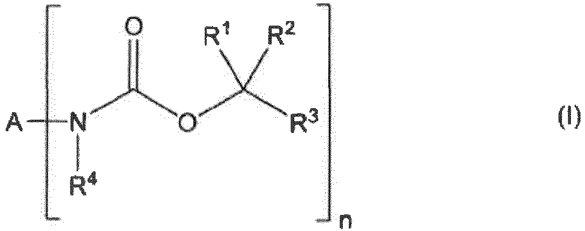 wobei
R¹ und R² unabhängig lineare oder verzweigte einwertige Kohlenwasserstoffradikale darstellen, die 1 bis 10 Kohlenstoffatome, bevorzugt 1 bis 5 Kohlenstoffatome aufweisen, die wahlweise ein oder mehrere Heteroatome und/oder multiple C-C Bindungen und/oder cycloaliphatische und/oder aromatische Anteile enthalten oder zusammen ein zweiwertiges Kohlenwasserstoffradikal darstellen, das 4 bis 10 Kohlenstoffatome, bevorzugt 4 bis 6 Kohlenstoffatome aufweist;
R³ ein lineares oder verzweigtes einwertiges Kohlenwasserstoffradikal darstellt, das 1 bis 20 Kohlenstoffatome, bevorzugt 1 bis 10 Kohlenstoffatome aufweist und wahlweise ein oder mehrere Heteroatome, insbesondere ein oder mehrere wahlweise substituierte tertiäre Alkylcarbamate und/oder multiple C-C Bindungen und/oder cycloaliphatische und/oder aromatische Anteile enthält;
R⁴ ein Wasserstoffradikal oder ein lineares oder verzweigtes einwertiges Kohlenwasserstoffradikal darstellt, das 1 bis 10 Kohlenstoffatome, bevorzugt 1 bis 5 Kohlenstoffatome aufweist, die wahlweise ein oder mehrere Heteroatome und/oder multiple C-C Bindungen und/oder cycloaliphatische und/oder aromatische Anteile enthalten oder zusammen mit A ein (n + 1)-wertiges Kohlenwasserstoffradikal darstellt, das 4 bis 40 Kohlenstoffatome, bevorzugt 4 bis 20 Kohlenstoffatome aufweist, die wahlweise ein oder mehrere Heteroatome und/oder cycloaliphatische Anteile enthalten;
A ein lineares oder verzweigtes n-wertiges Kohlenwasserstoffradikal darstellt, das 1 bis 20 Kohlenstoffatome aufweist, das wahlweise ein oder mehrere Heteroatome, insbesondere in Form von Ethersauerstoff oder Aminsauerstoff und wahlweise eine oder mehrere multiple C-C-Bindungen und/oder cycloaliphatische und/oder aromatische Anteile enthält oder zusammen mit R⁴ ein (n + 1)-wertiges Kohlenwasserstoffradikal darstellt, das 4 bis 40 Kohlenstoffatome, bevorzugt 4 bis 20 Kohlenstoffatome aufweist, das wahlweise ein oder mehrere Heteroatome und/oder cycloaliphatische Anteile enthält;
der Index n für einen Wert von 1 oder 2 oder 3 oder 4, bevorzugt 2 oder 3 steht.

2. Wärmedehnbare Zusammensetzung nach Anspruch 1, wobei der Index n gleich 2 ist und das Radikal A ein lineares oder verzweigtes zweiwertiges Kohlenwasserstoffradikal darstellt, das 2 bis 10 Kohlenstoffatome aufweist und das wahlweise ein oder mehrere Heteroatome insbesondere in Form von Ethersauerstoff enthält.

3. Wärmedehnbare Zusammensetzung nach Anspruch 1 oder 2, wobei das Carbamat **CA** ein Molekulargewicht zwischen 131 g/Mol und 750 g/Mol, bevorzugt zwischen 131 g/Mol und 500 g/Mol, noch bevorzugter zwischen 131 g/Mol und 450 g/Mol aufweist.

4. Wärmedehnbare Zusammensetzung nach irgendeinem der Ansprüche 1 bis 3, wobei mindesten 50 % des Carbamats **CA** sich unter Gasfreisetzung bei einer Temperatur zwischen 170 °C und 230 °C innerhalb von 10 min zersetzen.

5. Wärmedehnbare Zusammensetzung nach irgendeinem der Ansprüche 1 bis 4, wobei die Radikale R¹, R² und R³ Methylgruppen darstellen.

6. Wärmedehnbare Zusammensetzung nach irgendeinem der Ansprüche 1 bis 5, wobei der Stabilisator **S** in einer Menge zwischen 0,1 Gew.-% und 3 Gew.-%, bevorzugt zwischen 0,2 Gew.-% und 2 Gew.-%, noch bevorzugter zwischen 0,25 Gew.-% und 1 Gew.-%, auf das Gesamtgewicht der wärmedehnbaren Zusammensetzung bezogen, enthalten ist.

7. Wärmedehnbare Zusammensetzung nach irgendeinem der Ansprüche 1 bis 6, ferner einen Aktivator **AC** für das Carbamat **CA** umfassend, wobei der Aktivator **AC** mindestens eine Carbonsäure, bevorzugt eine Carbonsäure mit einem pKa-Wert zwischen 2,5 und 5, insbesondere Salicylsäure, umfasst.

8. Wärmedehnbare Zusammensetzung nach Anspruch 7, wobei das Molverhältnis von Carbamatfunktionen des Carbamats **CA** zu Carbonsäurefunktionen des Aktivators **AC** 1:1 beträgt.

9. Umlenkblech und/oder Verstärkungselement für hohle Strukturen, wobei das Element eine wärmedehnbare Zusammensetzung nach irgendeinem der Ansprüche 1 bis 8 und wahlweise einen Träger umfasst, auf den die wärmedehnbare Zusammensetzung abgesetzt oder befestigt ist.

10. Verwendung eines Carbamats **CA** nach Anspruch 1, bevorzugt in Kombination mit einem Aktivator **AC** als Treibmittel in wärmedehnbaren Zusammensetzungen.

11. Verwendung des Umlenkblechs und/oder Verstärkungselements nach Anspruch 9 zum Abdichten, Durchkreuzen oder Verstärken eines Hohlraums oder einer hohlen Struktur eines Landfahrzeugs, Wasserfahrzeugs oder Flugzeugs, bevorzugt eines Automobils und/oder eines Hohlraums eines Gebäudes, derart, dass die Übertragung von Geräusch, Vibrationen, Feuchtigkeit und/oder Hitze reduziert ist und/oder das Objekt, das den Hohlraum umgibt, mechanisch verstärkt ist.

12. Verfahren zum Abdichten, Durchkreuzen und/oder Verstärken eines Hohlraums oder einer hohlen Struktur, **dadurch gekennzeichnet, dass** ein Element, das eine wärmedehnbare Zusammensetzung nach irgendeinem der Ansprüche 1 bis 8 umfasst, in den Hohlraum oder die hohle Struktur eingeführt und daraufhin wärmegedehnt wird, derart, dass der Hohlraum oder die hohle Struktur mindestens teilweise durch die gedehnten Zusammensetzung gefüllt ist.

## Revendications

1. Composition thermiquement expansible, comprenant
a) au moins un agent gonflant chimique,
b) au moins un matériau thermoplastique,
c) facultativement au moins un stabilisant S,
dans laquelle
l'au moins un agent gonflant chimique comprend au moins un carbamate CA selon la formule (I), 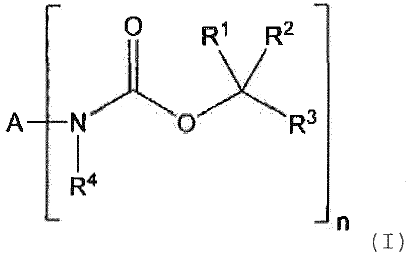 dans laquelle
R¹ et R² représentent indépendamment des radicaux hydrocarbonés monovalents linéaires ou ramifiés ayant 1 à 10 atomes de carbone, de préférence 1 à 5 atomes de carbone, qui contiennent facultativement un ou plusieurs hétéroatomes, et/ou liaisons multiples C-C, et/ou fragments cycloaliphatiques, et/ou fragments aromatiques, ou représentent conjointement un radical hydrocarboné divalent ayant 4 à 10 atomes de carbone, de préférence 4 à 6 atomes de carbone ;
R³ représente un radical hydrocarboné monovalent linéaire ou ramifié ayant 1 à 20 atomes de carbone, de préférence 1 à 10 atomes de carbone, et contient facultativement un ou plusieurs hétéroatomes, en particulier un ou plusieurs carbamates d'alkyle tertiaire facultativement substitué, et/ou liaisons multiples C-C, et/ou fragments cycloaliphatiques, et/ou fragments aromatiques ;
R⁴ représente un radical hydrogène ou un radical hydrocarboné monovalent linéaire ou ramifié ayant 1 à 10 atomes de carbone, de préférence 1 à 5 atomes de carbone, qui contient facultativement un ou plusieurs hétéroatomes, et/ou liaisons multiples C-C, et/ou fragments cycloaliphatiques, et/ou fragments aromatiques, ou, conjointement avec A, représente un radical hydrocarboné (n+1)-valent ayant 4 à 40 atomes de carbone, de préférence 4 à 20 atomes de carbone, qui contient facultativement un ou plusieurs hétéroatomes, et/ou fragments cycloaliphatiques ;
A représente un radical hydrocarboné n-valent linéaire ou ramifié ayant 1 à 20 atomes de carbone, qui contient facultativement un ou plusieurs hétéroatomes, en particulier sous la forme d'oxygène d'éther ou d'azote d'amine, et facultativement une ou plusieurs liaisons multiples C-C, et/ou un ou plusieurs fragments cycloaliphatiques, et/ou fragments aromatiques, ou, conjointement avec R⁴, représente un radical hydrocarboné (n+1-valent ayant 4 à 40 atomes de carbone, de préférence 4 à 20 atomes de carbone, qui contient facultativement un ou plusieurs hétéroatomes, et/ou fragments cycloaliphatiques ;
l'indice n désigne une valeur de 1 ou 2 ou 3 ou 4, de préférence 2 ou 3.

2. Composition thermiquement expansible selon la revendication 1, dans laquelle ledit indice n est égal à 2 et ledit radical A représente un radical hydrocarboné divalent linéaire ou ramifié ayant 2 à 10 atomes de carbone et qui contient facultativement un ou plusieurs hétéroatomes, en particulier sous la forme d'un oxygène d éther.

3. Composition thermiquement expansible selon la revendication 1 ou 2, dans laquelle ledit carbamate CA a un poids moléculaire compris entre 131 g/mol et 750 g/mol, de préférence entre 131 g/mol et 500 g/mol, plus préférablement entre 131 g/mol et 450 g/mol.

4. Composition thermiquement expansible selon l'une quelconque des revendications 1 à 3, dans laquelle au moins 50 % dudit carbamate CA se décompose avec libération de gaz à une température comprise entre 170 °C et 230 °C en 10 min.

5. Composition thermiquement expansible selon l'une quelconque des revendications 1 à 4, dans laquelle lesdits radicaux R¹, R² et R³ représentent des groupes méthyle.

6. Composition thermiquement expansible selon l'une quelconque des revendications 1 à 5, dans laquelle ledit stabilisant S est contenu en une quantité comprise entre 0,1 % en poids et 3 % % en poids, de préférence entre 0,2 % en poids et 2 % en poids, plus préférablement entre 0,25 % en poids et 1 % en poids, sur la base du poids total de la composition thermiquement expansible.

7. Composition thermiquement expansible selon l'une quelconque des revendications 1 à 6, comprenant en outre un activateur AC pour ledit carbamate CA, dans laquelle ledit activateur AC comprend au moins un acide carboxylique, de préférence un acide carboxylique avec une valeur pKa comprise entre 2,5 et 5, en particulier l'acide salicylique.

8. Composition thermiquement expansible selon la revendication 7, dans laquelle le rapport molaire des fonctions carbamate dudit carbamate CA aux fonctions acide carboxylique dudit activateur AC est 1:1.

9. Élément de déflecteur et/ou de renforcement pour structures creuses, ledit élément comprenant une composition thermiquement expansible selon l'une quelconque des revendications 1 à 8 et facultativement un support sur lequel ladite composition expansible est déposée ou fixée.

10. Utilisation d'un carbamate CA selon la revendication 1, de préférence en combinaison avec un activateur AC, en tant qu'agent gonflant dans des compositions thermiquement expansibles.

11. Utilisation de l'élément de déflecteur et/ou de renforcement selon la revendication 9 pour sceller, cloisonner ou renforcer une cavité ou structure creuse d'un véhicule terrestre, d'une embarcation ou d'un aéronef, de préférence une automobile, et/ou une cavité d'un bâtiment de sorte que la transmission du bruit, des vibrations, de l'humidité et/ou de la chaleur soit réduite, et/ou l'objet entourant ladite cavité soit mécaniquement renforcé.

12. Procédé pour sceller, cloisonner et/ou renforcer une cavité ou structure creuse, **caractérisé en ce qu'**un élément comprenant une composition thermiquement expansible selon l'une quelconque des revendications 1 à 8 est introduit dans ladite cavité ou structure creuse et ensuite thermiquement expansé de sorte que ladite cavité ou structure creuse soit au moins partiellement remplie par la composition expansée.
